(19) Europäisches Patentamt
European Patent Office
Office européen des brevets

(11) Publication number: **0 313 991 A2**

(12) **EUROPEAN PATENT APPLICATION**

(21) Application number: **88117500.4**

(51) Int. Cl.⁴: **C07D 239/34 , C09K 19/34**

(22) Date of filing: **20.10.88**

(30) Priority: **26.10.87 JP 269923/87**

(43) Date of publication of application: **03.05.89 Bulletin 89/18**

(84) Designated Contracting States: **AT BE CH DE FR GB IT LI LU NL SE**

(71) Applicant: **CHISSO CORPORATION**
**6-32, Nakanoshima 3-chome Kitaku**
**Osaka(JP)**

(72) Inventor: **Ohno, Kouji**
**27-2, Tatsumidai-higashi 3-chome**
**Ichiharashi Chiba-ken(JP)**
Inventor: **Ushioda, Makoto**
**27-2, Tatsumidai-higashi 3-chome**
**Ichiharashi Chiba-ken(JP)**
Inventor: **Saito, Shinichi**
**8890, Goi**
**Ichiharashi Chiba-ken(JP)**
Inventor: **Miyazawa, Kazutoshi**
**17, Tatsumidai-higashi 2-chome**
**Ichiharashi Chiba-ken(JP)**

(74) Representative: **Hansen, Bernd, Dr.rer.nat. et al**
**Hoffmann, Eitle & Partner Patentanwälte**
**Arabellastrasse 4 Postfach 81 04 20**
**D-8000 München 81(DE)**

(54) **5-Alkoxy-2-(4-alkoxyphenyl)pyrimidine, method for its preparation and use.**

(57) A novel smectic C liquid crystal compound useful as a component of ferroelectric liquid crystal materials capable of effecting a quick response is provided, which compound is a smectic C liquid crystalline 5-alkoxy-2-(4-alkoxyphenyl)pyrimidine compound expressed by the formula

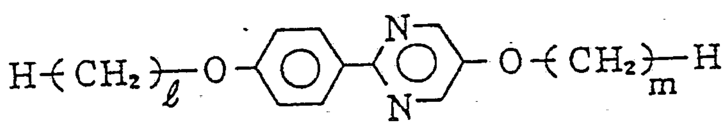

wherein ℓ represents an integer of 3 to 18 and m represents an integer of 6 to 18, but when ℓ represents 3 or 6, m represents 7 to 18.

**EP 0 313 991 A2**

**5-Alkoxy-2-(4-alkoxyphenyl)pyrimidine, method for its preparation and use.**

## BACKGROUND OF THE INVENTION

### 1. Field of the Invention

This invention relates to a novel smectic liquid crystal compound suitable for ferroelectric liquid crystal materials.

### 2. Description of the Related Art

At present, TN (Twisted Nematic) display mode has been most broadly employed for liquid crystal display elements. This TN liquid crystal display has a number of advantages such as low driving voltage, small electric consumption, etc., but it is inferior in the aspect of response rate to emissive mode display elements such as cathode ray tube display, electroluminescence display, plasma display, etc.

A novel TN mode display element having the twist angle increased to 180 - 270° has also been developed, but its response rate is still inferior. Various efforts for such an improvement have been made, but a TN mode display element having a high response rate has not yet been realized. However, a novel display mode having recently been extensively researched and using ferroelectric liquid crystals has a possibility of notably improving the response rate (Clark et al; Applied Phys. lett., 36. 899 (1980)). This mode utilizes chiral smectic phases such as chiral smectic C phase (hereinafter abbreviated to SC*) exhibiting ferroelectric properties. It has been known that phases exhibiting ferroelectric properties are not limited to SC* phase, but chiral smectic F, G, H, I phase and the like also exhibit ferroelectric properties.

Various specific features are required for ferroelectric liquid crystal materials used for ferroelectric liquid crystal display elements practically utilized, but at present, there is no single compound which satisfies all of these specific features; thus it is necessary to use ferroelectric liquid crystal compositions obtained by blending some liquid crystal compounds together or blending non-liquid crystal compounds therewith.

Further, ferroelectric liquid crystal compositions are not limited only to those composed only of ferroelectric liquid crystal compounds, but it has been reported in Japanese patent application laid-open No. Sho 60-36003/1985 that when at least one compound exhibiting ferroelectric liquid crystal phase is mixed with compound(s) or compositions exhibiting achiral smectic C, F, G, H, I phase or the like (hereinafter abbreviated to SC phase or the like) as base substance(s), it is possible to make the resulting mixture a ferroelectric liquid crystal composition as a whole. Further, it has also been reported that when at least one compound which is optically active but exhibits no ferroelectric liquid crystal phase is mixed with compound(s) or composition(s) exhibiting SC phase or the like as base substance(s), the resulting mixture is made into a ferroelectric liquid crystal composition as a whole (Mol. Cryst. Liq. Cryst. 89, 327 (1982)).

In a summary of these facts, it is seen that when at least one optically active compound, irrespective of whether it exhibits ferroelectric liquid crystal phase, is mixed with a base substance, it is possible to compose a ferroelectric liquid crystal composition.

As such base substances, various compounds exhibiting achiral smectic liquid crystal phase of SC or the like may be used, and practically, liquid crystal compound(s) or mixture(s) exhibiting smectic phase (abbreviated to SC phase) within a broad temperature range including room temperature are preferred. Examples of components of these smectic C liquid crystal mixtures are liquid crystal compounds of phenyl benzoates, Schiff's bases, biphenyls, phenylpyridines, 5-alkyl-2-(4-alkoxyphenyl)pyrimidines, etc.

For examples, Japanese patent application laid-open No. Sho 61-291679/1986 and WO 86/06401 disclose that a mixture of a 5-alkyl-2-(4-alkoxyphenyl)pyrimidine compound with an optically active compound is used as a ferroelectric liquid crystal material.

West German patent publication No. DT 2257588 discloses that a nematic liquid crystalline pyrimidine derivative expressed by the formula

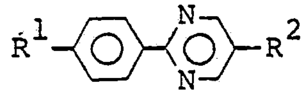

wherein $R^1$ and $R^2$ each represent an alkyl or alkoxy group each of 1 to 12 carbon atoms and a nematic liquid crystal mixture comprising the derivative are applied to an electrooptical device. Concretely disclosed example of the pyrimidine derivative of the above formula is only 5-butoxy-2-(4-pentyloxyphenyl)pyrimidine (abbreviated to compound A) and this compound is described therein to be a compound exhibiting nematic phase.

Thereafter, a handbook, Flüssige Kristalle in Tabellen II (1984) describes a report of the inventors of the above DT that a concrete compound, 5-hexyloxy-2-(4-hexyloxyphenyl)pyrimidine (abbreviated to compound B) is additionally listed and the phase transition points (°C) of these compounds are described as follows:

| | C | SA | N | I |
|---|---|---|---|---|
| Compound A | • 82 | - | • 88 | • |
| Compound B | • 62 | • 68 | • 89 | • |

Thus, it has not been considered that the presence of smectic C phase in compounds having the core structure of 5-alkoxy-2-(4-alkoxyphenyl)pyrimidine might be expected from the above disclosures.

## SUMMARY OF THE INVENTION

The object of the present invention is to provide a novel smectic C liquid crystal compound useful as a component of ferroelectric liquid crystal materials capable of effecting a high-speed response.

The present invention resides in a smectic C liquid crystalline 5-alkoxy-2-(4-alkoxyphenyl)pyrimidine compound expressed by the formula

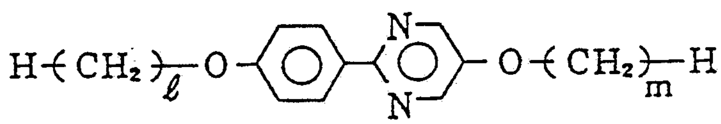

( I )

wherein ℓ represents an integer of 3 to 18 and m represents an integer of 6 to 18, but when ℓ represents 3 or 6, m represents 7 to 18.

## DETAILED DESCRIPTION OF PREFERRED EMBODIMENTS

The compound of the present invention is characterized by having a smectic C liquid crystal phase. Unlike liquid crystal phases such as smectic A phase, cholesteric phase, nematic phase, etc., smectic C liquid crystal phase is a liquid crystal phase capable of exhibiting ferroelectric properties when a suitable quantity of an optically active substance is mixed therewith, and also SC phase is present in the highest temperature region among those of liquid crystal phases exhibiting ferroelectric properties. In other words, liquid crystals having SC phase are most important as compared with liquid crystals having other tilted smectic phases, from the viewpoint of a component of ferroelectric liquid crystal materials.

Representative compounds of the present invention and their phase transition points are shown in Table 1. In Table 1, C, SC, SA, N and I represent crystalline phase, smectic C phase, smectic A phase, nematic phase and isotropic liquid phase, respectively, and the symbols • and - below the above-mentioned symbols represent the presence and absence of the phases of these symbols, respectively.

Table 1

| No. | In formula (I) | | Phase transition points (℃) | | | | |
|---|---|---|---|---|---|---|---|
| | ℓ | m | C | SC | SA | N | I |
| 1 | 3 | 7 | • 68.6 | (• 65.0) | • 78.7 | • 83.6 | • |
| 2 | 〃 | 8 | • 49.6 | • 70.5 | • 88.2 | • 88.7 | • |
| 3 | 〃 | 9 | • 43.7 | • 72.0 | • 89.4 | — | • |
| 4 | 〃 | 10 | • 45.6 | • 71.0 | • 92.6 | — | • |
| 5 | 〃 | 11 | • 41.9 | • 68.0 | • 93.0 | — | • |
| 6 | 〃 | 12 | • 43.4 | • 61.6 | • 94.1 | — | • |
| 7 | 4 | 7 | • 53.4 | • 75.1 | • 82.4 | • 92.1 | • |
| 8 | 〃 | 8 | • 54.4 | • 84.0 | • 94.7 | • 96.6 | • |
| 9 | 〃 | 9 | • 44.4 | • 87.7 | • 96.6 | — | • |
| 10 | 〃 | 10 | • 41.8 | • 90.0 | • 99.4 | — | • |
| 11 | 〃 | 11 | • 42.3 | • 89.0 | • 99.8 | — | • |
| 12 | 〃 | 12 | • 41.8 | • 88.4 | • 101.0 | — | • |
| 13 | 5 | 6 | • 62.0 | • 65.9 | • 76.6 | • 92.7 | • |
| 14 | 〃 | 7 | • 54.4 | • 77.4 | • 84.2 | • 91.3 | • |
| 15 | 〃 | 8 | • 50.2 | • 85.9 | • 93.9 | • 94.8 | • |
| 16 | 〃 | 9 | • 66.7 | • 90.0 | • 95.0 | — | • |
| 17 | 〃 | 10 | • 41.4 | • 93.9 | • 97.2 | — | • |
| 18 | 〃 | 11 | • 51.4 | • 95.9 | • 98.2 | — | • |
| 19 | 〃 | 12 | • 41.7 | • 96.2 | • 98.6 | — | • |
| 20 | 6 | 7 | • 45.8 | • 80.6 | • 84.7 | • 94.9 | • |
| 21 | 〃 | 8 | • 42.8 | • 89.8 | • 96.6 | • 98.7 | • |
| 22 | 〃 | 9 | • 49.9 | • 94.4 | • 97.9 | — | • |
| 23 | 〃 | 10 | • 43.8 | • 98.7 | • 100.3 | — | • |

Table 1 (-continued)

| № | In formula (I) | | Phase transition points | | | | (℃) |
|---|---|---|---|---|---|---|---|
| | ℓ | m | C | SC | SA | N | I |
| 24 | 6 | 11 | • 55.4 | • 100.4 | • 101.0 | — | • |
| 25 | 〃 | 12 | • 52.2 | • 102.2 | — | — | • |
| 26 | 7 | 6 | • 60.8 | • 68.2 | • 79.4 | • 94.6 | • |
| 27 | 〃 | 7 | • 59.2 | • 79.9 | • 87.6 | • 93.6 | • |
| 28 | 〃 | 8 | • 51.6 | • 87.2 | • 96.4 | • 97.1 | • |
| 29 | 〃 | 9 | • 56.0 | • 94.9 | • 97.8 | — | • |
| 30 | 〃 | 10 | • 55.6 | • 99.6 | • 100.3 | — | • |
| 31 | 〃 | 11 | • 67.4 | • 100.1 | — | — | • |
| 32 | 〃 | 12 | • 54.8 | • 100.3 | — | — | • |
| 33 | 8 | 6 | • 59.3 | • 73.0 | • 80.0 | • 96.2 | • |
| 34 | 〃 | 7 | • 53.4 | • 81.5 | • 88.3 | • 95.4 | • |
| 35 | 〃 | 8 | • 47.7 | • 90.6 | • 98.7 | • 99.4 | • |
| 36 | 〃 | 9 | • 48.2 | • 96.4 | • 99.0 | — | • |
| 37 | 〃 | 10 | • 51.7 | • 101.7 | • 102.1 | — | • |
| 38 | 〃 | 11 | • 59.9 | • 101.7 | — | — | • |
| 39 | 〃 | 12 | • 57.1 | • 102.9 | — | — | • |
| 40 | 9 | 6 | • 61.6 | • 68.9 | • 83.2 | • 93.7 | • |
| 41 | 〃 | 7 | • 55.2 | • 78.7 | • 89.6 | • 93.6 | • |
| 42 | 〃 | 8 | • 55.1 | • 87.5 | • 98.2 | — | • |
| 43 | 〃 | 9 | • 60.1 | • 95.3 | • 98.4 | — | • |
| 44 | 〃 | 10 | • 52.5 | • 101.1 | — | — | • |
| 45 | 〃 | 11 | • 62.0 | • 101.0 | — | — | • |
| 46 | 〃 | 12 | • 60.3 | • 100.3 | — | — | • |

Table 1 (-continued)

| No. | In formula (I) ℓ | In formula (I) m | Phase transition points (℃) C | SC | SA | N | I |
|---|---|---|---|---|---|---|---|
| 47 | 10 | 6 | • 62.3 | • 71.6 | • 83.8 | • 93.6 | • |
| 48 | 〃 | 7 | • 50.4 | • 79.7 | • 90.1 | • 93.6 | • |
| 49 | 〃 | 8 | • 50.4 | • 88.0 | • 98.5 | — | • |
| 50 | 〃 | 9 | • 52.3 | • 96.2 | • 99.0 | — | • |
| 51 | 〃 | 10 | • 52.7 | • 101.4 | — | — | • |
| 52 | 〃 | 11 | • 62.9 | • 101.2 | — | — | • |
| 53 | 〃 | 12 | • 65.4 | • 102.8 | — | — | • |
| 54 | 11 | 6 | • 69.3 | (• 69.0) | • 86.2 | • 91.8 | • |
| 55 | 〃 | 7 | • 58.2 | • 77.0 | • 90.1 | • 91.9 | • |
| 56 | 〃 | 8 | • 56.0 | • 84.9 | • 97.1 | — | • |
| 57 | 〃 | 9 | • 56.2 | • 92.7 | • 96.1 | — | • |
| 58 | 〃 | 10 | • 53.1 | • 100.6 | — | — | • |
| 59 | 〃 | 11 | • 69.8 | • 99.8 | — | — | • |
| 60 | 〃 | 12 | • 65.6 | • 101.0 | — | — | • |
| 61 | 〃 | 16 | • 71.3 (1) | • 100.9 | — | — | • |
| 62 | 12 | 6 | • 70.3 | • 70.7 | • 86.2 | • 91.4 | • |
| 63 | 〃 | 7 | • 57.1 | • 77.2 | • 89.4 | • 90.9 | • |
| 64 | 〃 | 8 | • 51.8 | • 85.0 | • 96.4 | — | • |
| 65 | 〃 | 9 | • 53.8 | • 93.5 | • 96.9 | — | • |
| 66 | 〃 | 10 | • 54.6 | • 100.3 | — | — | • |
| 67 | 〃 | 11 | • 59.5 | • 100.7 | — | — | • |
| 68 | 〃 | 12 | • 63.7 | • 104.3 | — | — | • |
| 69 | 〃 | 16 | • 71.7 (2) | • 99.0 | — | — | • |

(1): Unidentified smectic phase ($S_1$) transits into SC phase at 75.6°C.

(2): Unidentified smectic phase ($S_1$) transits into SC phase at 73.7°C.

As seen from Table 1, as to the pyrimidine compound of the formula (I), the larger the chain length of the alkyloxy group bonded to the pyrimidine ring, the greater the smectic C properties. Further, surprisingly enough, compounds having a larger alkyloxy group chain length include a number of liquid crystals exhibiting no nematic phase. This is a fact almost unanticipated from he disclosures of the above-mentioned DT and Handbook.

The present inventors have already found a 5-alkoxy-2-(4-alkylphenyl)pyrimidine compound having smectic C liquid crystal properties (Japanese patent application No. Sho 62-137884/1987). This compound has been found noting the specific properties of 5-alkoxypyrimidine compounds. In the case of the compound, it has been seen that as its alkyl chain length increases, another smectic phase is liable to appear on the lower temperature side relative to SC phase; thus the SC phase region tends to be narrowed. This drawback has been considerably overcome by the smectic C liquid crystalline 5-alkoxy-2-(4-alkoxyphenyl)pyrimidine compound of the present invention. Namely, according to the present invention, a pyrimidine compound having more improved the thermodynamic stability of SC phase is provided and a more practical smectic C liquid crystal mixture and a ferroelectric smectic C liquid crystal mixture are obtained.

When the smectic C liquid crystalline 5-alkoxy-2-(4-alkoxyphenyl)pyrimidine of the present invention is mixed alone or in the form of a smectic C mixture consisting of a plurality thereof with at least one optically active compound, it is possible to compose a ferroelectric liquid crystal material, and also the resulting chiral smectic C liquid crystal material exhibits a very high response rate.

The compound of the present invention may be prepared for example through the following preparation route:

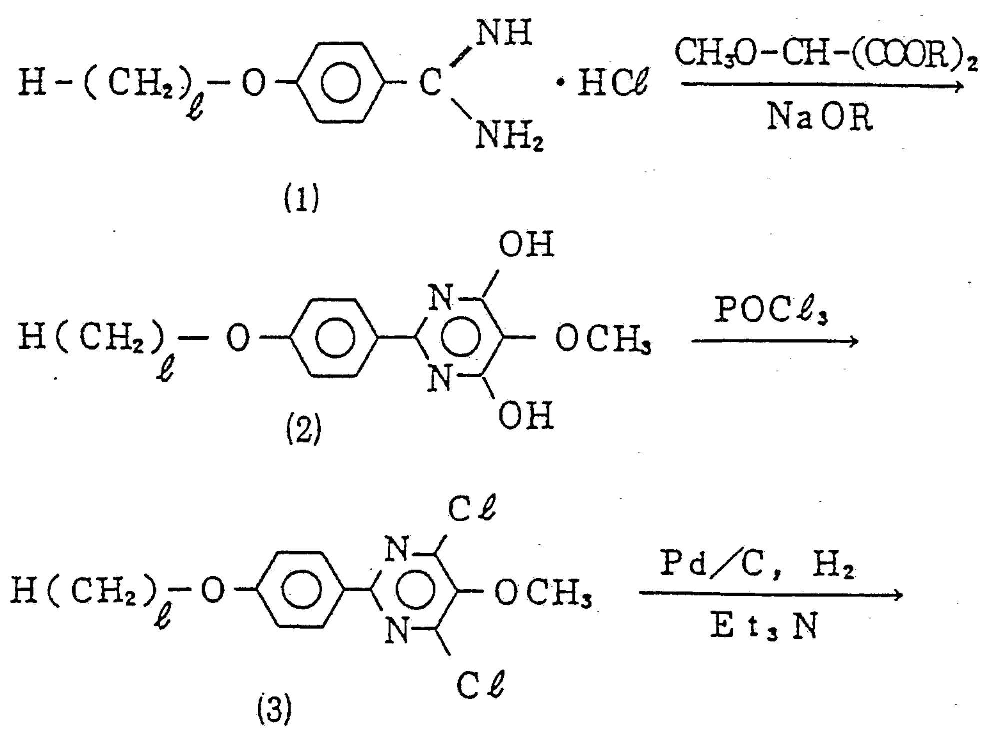


(1)

(2)

(3)

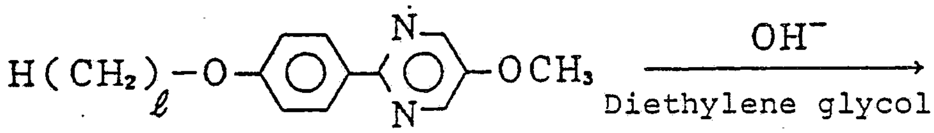


(4)

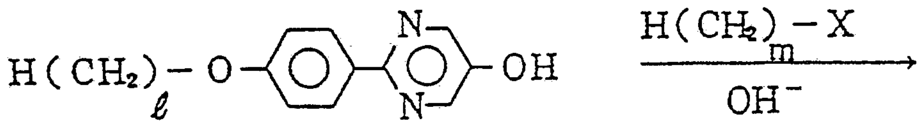


(5)

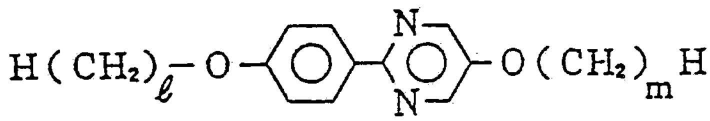

(I)

wherein R represents an alkyl group such as methyl group, ethyl group, etc. and X represents a leaving group such as chlorine, bromine, iodine, p-toluenesulfonyloxy group, benzenesulfonyloxy group, methanesulfonyl group, etc.

Namely, a p-alkylbenzamidine hydrochloride (1) is reacted with a methoxymalonic acid diester in the presence of a sodium alcoholate to obtain a diol (2), which is then halogen-substituted by means of a halogenating agent such as phosphorus oxychloride to obtain a compound (3), which is then dehalogenated in the presence of a base to obtain a compound (4), which is then heat-treated in diethylene glycol in the presence of an alkali to obtain a compound (5), which is etherified to obtain a compound (I). Further, the compound of the formula (I) may also be prepared through the following route:

(6)

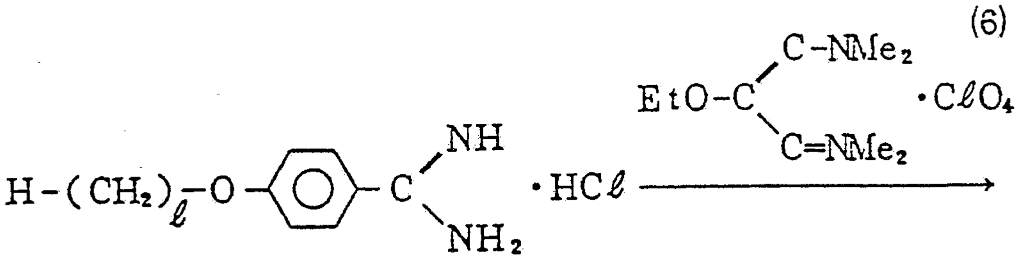

(1)

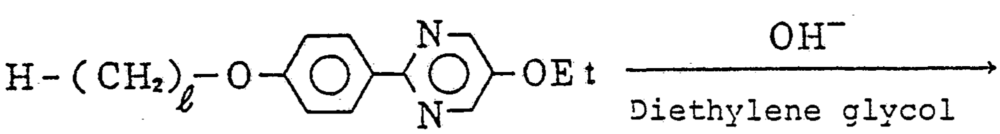


(7)

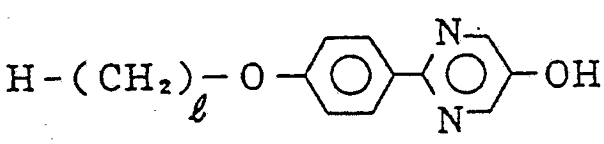

(5)

Namely, 1,3-bis(dimethylamino)-2-ethoxytrimethinium perchlorate (6) disclosed in a literature (Collection Chechoslov. Chem. Commun., 38 (1973), 1168) is reacted with a p-alkylbenzamidine hydrochloride (1) in the presence of a sodium alcoholate to prepare a compound (7), which is then alkali-treated to obtain a compound (5). The subsequent step is carried out in the same manner as in the last step of the aforementioned route to obtain the compound (I).

Further, the compound (I) may also be prepared as follows:

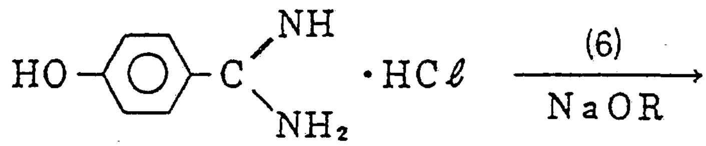


(8)

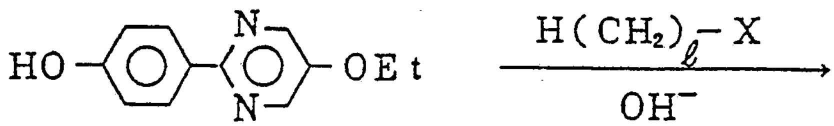


(9)

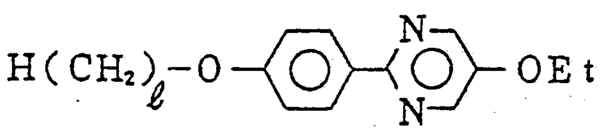

(7)

Namely, the compound (6) is reacted with a p-hydroxybenzamidine hydrochloride (8) in the presence of a sodium alcoholate to prepare a compound (9), which is then etherified to obtain a compound (7). The subsequent steps are carried out in the same manner as in the aforementioned route to obtain the compound (I)

The present invention will be described in more detail by way of Example .

## Example 1

Preparation of 5-heptyloxy-2-(4-octyloxyphenyl)pyrimidine

### (A) Preparation of 5-ethoxy-2-(4-hydroxyphenyl)pyrimidine:

To a solution of sodium methoxide (194.6 g) in methanol (2,000 mℓ) were added p-hydroxybenzamidine hydrochloride (200 g) and 1,3-bis(dimethylamino)-2-ethoxytrimethinium perchlorate (313.5 g) prepared in the same manner as disclosed in the above-mentioned literature, followed by heating the mixture under reflux for 6.5 hours, allowing the reaction mixture to cool down, adding water and acetic acid and filtering off deposited crystals to obtain 5-ethoxy-2-(4-hydroxyphenyl)pyrimidine (205 g). M.P.: 201.5 - 202.3 °C.

### (B) Preparation of 5-ethoxy-2-(4-octyloxyphenyl)pyrimidine:

5-Ethoxy-2-(4-hydroxyphenyl)pyrimidine (40 g) was dissolved in ethanol (340 mℓ), followed by adding potassium hydroxide (11.2 g) and octyl bromide (35.8 g), heating the mixture under reflux for 3 hours,

distilling off ethanol (about 300 mℓ) from the reaction mixture, adding toluene and water, extracting the objective product into the resulting toluene layer, washing the toluene layer with 2N-NaOH solution and then with water till the washing water became neutral, distilling off toluene and low boiling substances from the toluene solution and recrystallizing the residue from ethanol solvent to obtain 5-ethoxy-2-(4-octyloxyphenyl)-pyrimidine (46.9 g). The phase transition points of this product were as follows:

$$C \xleftrightarrow{86.3^\circ C} N \xleftrightarrow{93.0^\circ C} I$$

(C) Preparation of 5-hydroxy-2-(4-octyloxyphenyl)pyrimidine:

5-Ethoxy-2-(4-octyloxyphenyl)pyrimidine (48 g) was dissolved in diethylene glycol (300 mℓ), followed by adding NaOH (58.4 g), agitating the mixture at 220 °C for 3 hours, allowing it to cool down, adding water and acetic acid, filtering off deposited crystals and recrystallizing from ethanol to obtain 5-hydroxy-2-(4-octyloxyphenyl)pyrimidine (29 g).

(D) Preparation of 5-heptyloxy-2-(4-octyloxyphenyl)pyrimidine:

5-Hydroxy-2-(4-octyloxyphenyl)pyrimidine (4 g) was dissolved in ethanol (20 mℓ), followed by adding KOH (0.8 g) and heptyl bromide (2.4 g), heating the mixture under reflux for 6 hours, allowing it to cool down, adding toluene and water, washing the resulting separated organic layer with 2N-NaOH aqueous solution and then with water till the washing water became neutral, distilling off toluene and low boiling substances from the toluene layer and recrystallizing the residue from ethanol to obtain 5-heptyloxy-2-(4-octyloxyphenyl)pyrimidine (1.5 g). This product exhibited the following phase transition points:

$$C \xleftrightarrow{53.4^\circ C} SC \xleftrightarrow{81.5^\circ C} SA \xleftrightarrow{88.3^\circ C} N \xleftrightarrow{95.4^\circ C} I$$

**Claims**

1. A smectic C liquid crystalline 5-alkoxy-2-(4-alkoxyphenyl)pyrimidine compound expressed by the formula

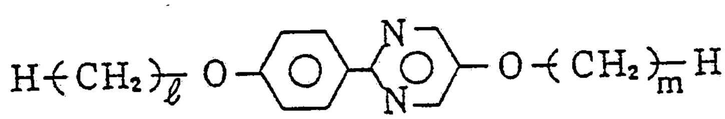

wherein ℓ represents an integer of 3 to 18 and m represents an integer of 6 to 18, but when represents 3 or 6, m represents 7 to 18.

2. A smectic C liquid crystalline 5-alkoxy-2-(4-alkoxyphenyl)pyrimidine compound according to claim 1 wherein ℓ represents an integer of 3 to 12 and m represents an integer of 6 to 12.

3. A method of production of a smectic C liquid crystalline 5-alkoxy-2-(4-alkoxy-phenyl) pyrimidine compound as claimed in claim 1
**characterized** by
the following preparation steps
a) reacting a p-alkylbenzamidine hydrochloride of the general formula (1)

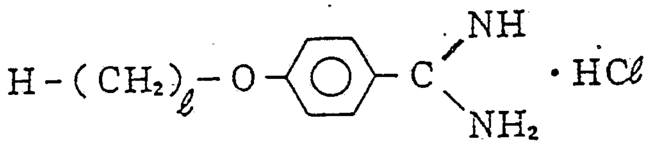

(1)

wherein 1 is an integer of 3 to 18 with the methoxymalonic acid diester of the formula $CH_3O\text{-}CH\text{-}(COOR)_2$ in the presence of a sodium alcoholate NaOR, wherein R represents an alkyl group, to obtain a diol of the formula (2)

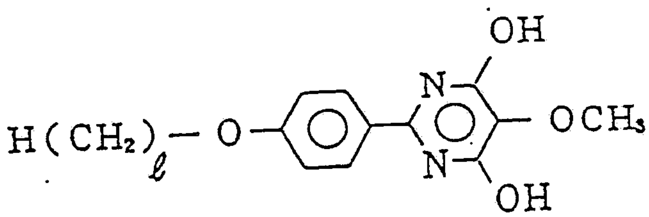

(2)

b) halogen-substituting the diol (2) by means of a halogenating agent such as phosphorus oxychloride to obtain a compound with the formula (3)

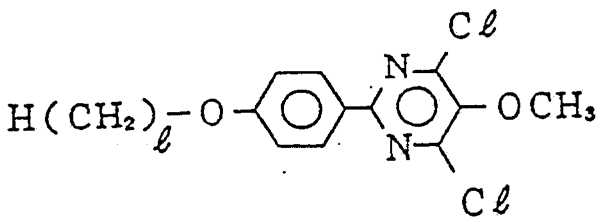

(3)

c) dehalogenating the compound (3) in the presence of a base to obtain a compound with the formula (4)

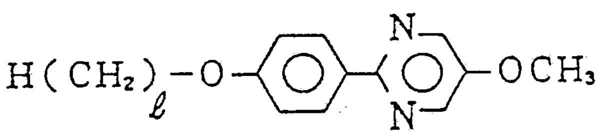

(4)

d) heat-treating the compound (4) in diethylene glycol in the presence of an alkali to obtain a compound with the formula (5)

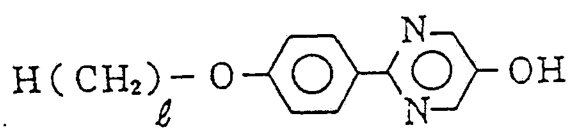

(5)

e) etherifying the compound (5) by means of a compound with the formula $H(CH_2)_m\text{-}X$ wherein m represents an integer of 6 to 18 and X represents a leaving group such as chlorine, bromine, iodine, p-toluenesulfonyloxy group, benzenesulfonyloxy group, methanesulfonyl group, to obtain a compound with the formula (I)

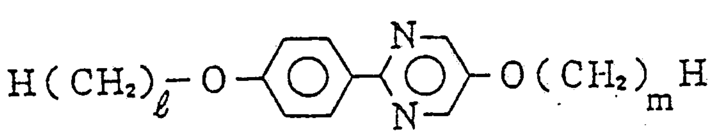

(I)

4. A method for the production of a smectic C liquid crystalline 5-alkoxy-2-(4-alkoxy-phenyl)pyrimidine compound as claimed in claim 1
**characterized** by
the following preparation steps:
a) reacting of a p-alkylbenzamidine hydrochloride of the formula (1)

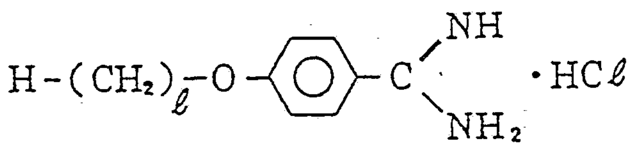

(1)

wherein 1 is an integer of 3 to 18 with 1,3-bis(dimethylamino)-2-ethoxytrimethinium perchlorate in the presence of a sodium alcoholate to prepare a compound with the formula (7)

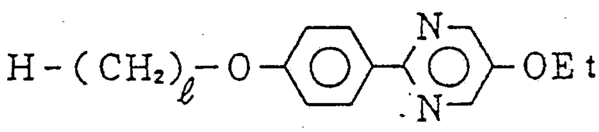

(7)

b) alkali-treating the compound (7) to obtain a compound with the formula (5)

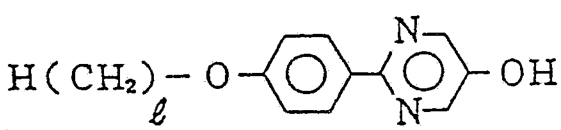

(5)

c) etherifying the compound (5) by means of the compound with the formula $H(CH_2)_m$-X wherein m represents an integer of 6 to 18 and X represents a leaving group such as chlorine, bromine, iodine, p-toluenesulfonyloxy group, benzenesulfonyloxy group, methanesulfonyl group, to obtain a compound with the formula (I)

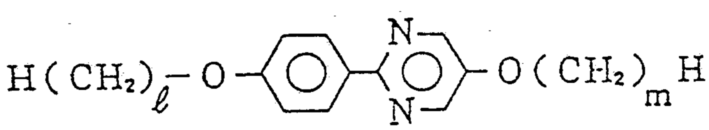

(I)

5. A method for production of a smectic C liquid crystalline 5-alkoxy-2-(4-alkoxy-phenyl)pyrimidine compound as claimed in claim 1
**characterized** by
the following preparation steps:
a) reacting of p-hydroxybenzamidine hydrochloride of the formula (8)

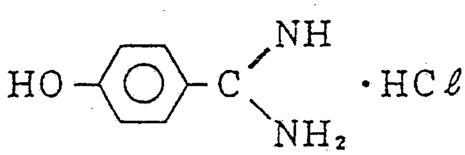

(8)

with 1,3-bis(dimethylamino)-2-ethoxytrimethinium perchlorate in the presence of a sodium alcoholate to prepare a compound with the formula (9)

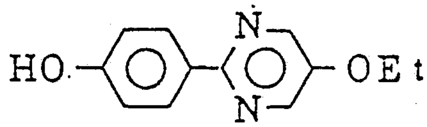

(9)

b) etherifying the compound (9) by means of a compound with the formula $H(CH_2)_1$-X wherein 1 represents an integer of 3 to 18 and X represents a leaving group such as chlorine, bromine, iodine, p-toluenesulfonyloxy group, benzenesulfonyloxy group, methanesulfonyl group, to obtain a compound with the formula (7)

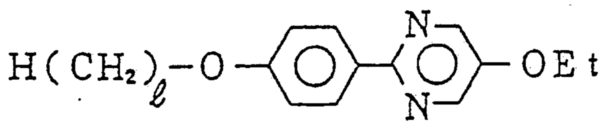

(7)

c) alkali-treating the compound (7) to obtain a compound with the formula (5)

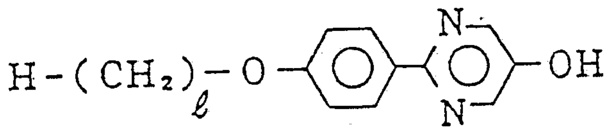

(5)

d) etherifying the compound (5) by means of a compound with the formula $H(CH_2)_m$-X wherein m is an integer of 6 to 18 and X represents a leaving group such as chlorine, bromine, iodine, p-toluenesulfonyloxy group, benzenesulfonyloxy group, methanesulfonyl group to obtain the compound with the formula (I)

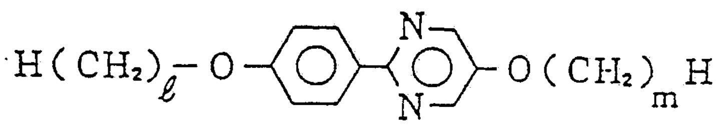

(I)

6. Use of a smectic C liquid crystalline 5-alkoxy-2-(4-alkoxy-phenyl)pyrimidine compound as claimed in claim 1 as a component of ferroelectric liquid crystal materials.